# FASCICULE DE BREVET EUROPEEN

(11) **EP 1 214 010 B1**
(45) Date de publication et mention de la délivrance du brevet: **24.11.2004**
(21) Numéro de dépôt: 00964317.2
(22) Date de dépôt: 19.09.2000
(51) Int. Cl.: A61B 19/08

(54) **DISPOSITIF DE VISIONNAGE CHIRURGICAL A ECRAN STERILISABLE**
CHIRURGISCHER BILDANZEIGEEINRICHTUNG MIT STERILISIERBAREM BILDSCHIRM
SURGICAL VIEWING DEVICE WITH DISPLAY SCREEN CAPABLE OF BEING STERILISED

(30) Priorité: 20.09.1999 FR 9911734
(43) Date de publication de la demande: 19.06.2002
(73) Titulaire: Barthes, Michel, 78110 Le Vesinet (FR)
(72) Inventeur: Barthes, Michel, 78110 Le Vesinet (FR)
(74) Mandataire: Martin, Jean-Jacques
(86) Numéro de dépôt international: PCT/FR2000/002591
(87) Numéro de publication internationale: WO 2001/021086

(56) Documents cités:
- WO-A-98/02107
- WO-A-98/03013
- WO-A-98/27561
- DE-A- 19 907 642
- GB-A- 2 064 879
- US-A- 4 894 748
- US-A- 4 963 693
- US-A- 5 111 222
- PATENT ABSTRACTS OF JAPAN vol. 014, no. 370 (C-0747), 10 août 1990 (1990-08-10) & JP 02 136123 A (OLYMPUS OPTICAL CO LTD), 24 mai 1990 (1990-05-24)
- PATENT ABSTRACTS OF JAPAN vol. 1996, no. 10, 31 octobre 1996 (1996-10-31) & JP 08 150114 A (FUKUDA DENSHI CO LTD), 11 juin 1996 (1996-06-11)
- PATENT ABSTRACTS OF JAPAN vol. 1997, no. 02, 28 février 1997 (1997-02-28) & JP 08 280610 A (OLYMPUS OPTICAL CO LTD), 29 octobre 1996 (1996-10-29)

## Description

L'invention concerne les dispositifs d'observation d'un champ chirurgical, et notamment de tels dispositifs couplés à un endoscope.

De manière traditionnelle, de tels dispositifs sont constitués d'une caméra endoscopique que le chirurgien guide manuellement dans le corps du patient, et d'un moniteur vidéo placé à quelques mètres, sur lequel est affichée l'image prélevée par la caméra.

Le chirurgien utilise un instrument chirurgical dont il guide l'extrémité d'attaque apparaissant sur l'écran.

De tels écrans de télévision ordinaires sont difficilement stérilisables de sorte que les praticiens se sont longtemps contentés de maintenir ces écrans à l'écart du patient.

On a proposé dans le document WO 98 02107 un cache stérile pour écran plat, consistant simplement en une enveloppe flexible ayant sensiblement la forme de l'écran et se prolongeant par un manchon autour d'une partie d'un cordon d'alimentation et d'échange de signaux de l'écran.

On propose dans ce document de refermer cette enveloppe autour du cordon et d'aspirer en permanence l'air intérieur de cette enveloppe afin de plaquer celle-ci contre l'écran.

Selon une variante, ce document propose de placer un cache rigide autour de l'écran, comportant des orifices de connexion prévus pour être hermétiquement fermés lorsque connectés.

Dans cette variante, on obtient, après connexion, une enveloppe totalement étanche qui permet de stériliser l'ensemble par immersion sans devoir extraire l'écran du cache.

Un tel dispositif présente toutefois un lourd inconvénient, car sa stérilité est difficile à assurer.

L'étanchéité de l'ensemble repose entièrement sur l'étanchéité entre le cache et les connecteurs des câbles. Cette étanchéité est difficile à garantir, notamment du fait que les câbles sont souvent déplacés ou tirés et que la connexion est soumise à des forces élevées. L'herméticité entre l'extérieur stérile et l'intérieur non stérile de l'enceinte repose donc sur des moyens particulièrement fragiles.

En outre, le cache rigide doit pouvoir être ouvert et comprend pour cela au moins deux parties désolidarisables l'une de l'autre. Une étanchéité complète à la jonction entre ces deux pièces ne peut être garantie pour un nombre indéfini de stérilisations de l'enceinte, et il prend place une communication d'air entre l'intérieur et l'extérieur du cache au niveau de cette jonction.

Un tel dispositif ne peut donc pas être considéré comme fiable.

Le but principal de l'invention est de pallier cet inconvénient.

Pour cela, l'invention propose un dispositif pour observer un champ chirurgical, comprenant un moniteur vidéo plat et une enceinte qui est stérilisable et qui forme barrière de stérilité fermée de manière étanche autour du moniteur, caractérisé en ce que l'enceinte présente une étanchéité suffisante pour conserver une sur- ou sous-pression interne appliquée à la fermeture de l'enceinte et en ce que l'enceinte est munie d'un capteur de pression interne apte à activer des moyens d'alerte en cas de disparition de la sur- ou sous-pression interne, le dispositif assemblé étant stérilisable à l'infini et utilisable dans des salles stériles de toutes natures, par exemple médicale ou en laboratoire.

D'autres caractéristiques, buts et avantages de l'invention apparaîtront à la lecture de la description détaillée qui va suivre, faite en référence aux figures annexées sur lesquelles :

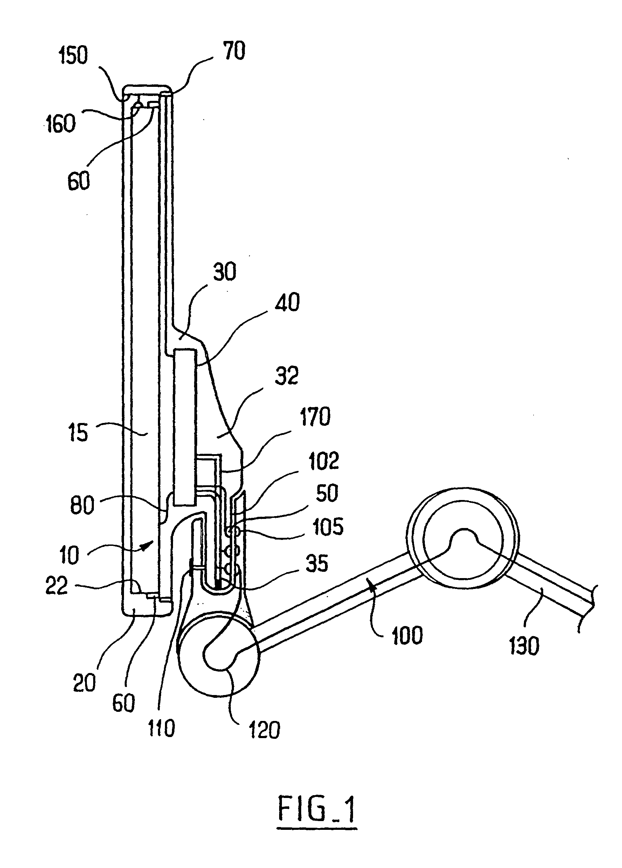

- la figure 1 présente un dispositif d'observation chirurgicale selon l'invention, en coupe transversale ;
- la figure 2 présente le même dispositif selon une même coupe, en vue éclatée.

Le présent dispositif d'observation se compose d'un ensemble d'affichage vidéo 10 et d'un bras articulé 100 formant support pour l'ensemble d'affichage 10.

L'ensemble d'affichage 10 est constitué de deux ensembles principaux, que sont un moniteur vidéo enfermé dans une enceinte étanche.

Le moniteur vidéo se présente sous la forme d'un écran à cristaux liquides 15 et d'un module électronique 40 relié à cet écran par une liaison filaire 80.

L'écran 15 est ici un écran à cristaux liquides (LCD) à matrice active, de type à micro-pointes, de technologie plasma, ou de technologie sans transistor type DEL (Digital Electronic Luminescence).

Dans ce mode de réalisation, ces deux éléments 15 et 40 sont maintenus en position l'un par rapport à l'autre par l'enceinte étanche.

Pour cela, cette dernière est formée de deux pièces 20 et 30, chacune de forme générale plane et rectangulaire et de dimensions légèrement supérieures à celles de l'écran 15.

L'élément 30 vient recouvrir l'ensemble de la face arrière de l'écran 15. Il présente pour cela une partie courante de forme globalement rectangulaire et quatre bords rectilignes longeant les quatre bords de l'écran 15.

Dans sa partie centrale, ce plateau arrière 30 s'étend en éloignement de l'écran 15 sous la forme d'une ondulation en forme de bosse, dont la partie concave dirigée vers l'écran 15 forme à l'arrière de celui-ci une cavité de réception pour le module électronique 40, et dont la partie convexe arrière se prolonge en un manche 35 destiné à être enfiché dans le support 100.

Plus précisément, la cavité interne formée par ce bossage 30 présente une forme parallélépipédique complémentaire de celle du module 40. Une fois le module 40 en place dans la cavité interne de ce bossage 30, et l'écran 15 relié au module 40, l'écran 15 reçoit par sa face avant la pièce 20 en forme générale de récipient parallépipédique dont le fond forme le plateau avant de l'écran 15 et dont les quatre bords relevés viennent recouvrir les quatre bords de l'écran 15.

Ces quatre bords sont assez élevés pour venir également recouvrir les quatre bords libres du plateau arrière 30 dépassant les bords de l'écran 15 et coopérer avec ceux-ci, de sorte que les deux éléments 20 et 30 forment une enceinte fermée autour de l'écran 15 et du module 40.

Pour fournir une étanchéité particulièrement fiable de l'ensemble, on dispose entre ces deux éléments 20 et 30, notamment à leur jonction de bordure, un joint en Téflon référencé 70.

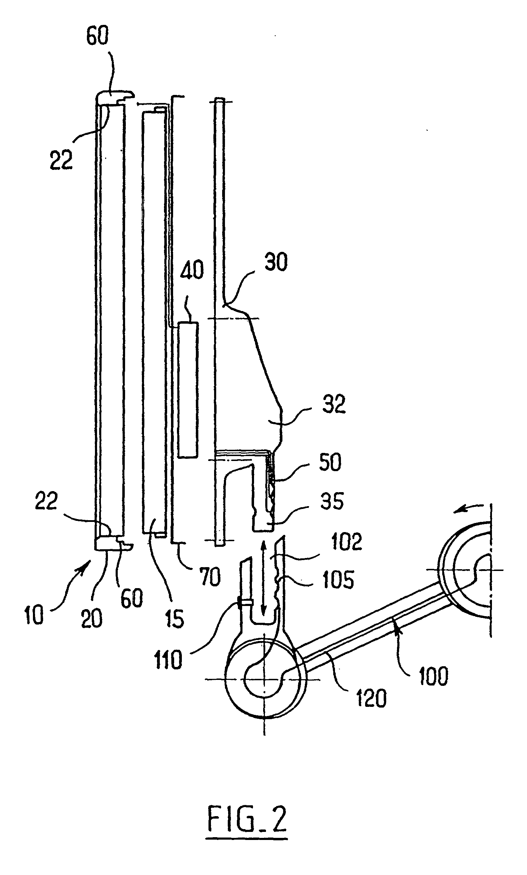

Comme on peut le voir sur la vue éclatée de la figure 2, ce joint 70 est ici une feuille qui recouvre l'ensemble de la face avant du plateau arrière 30 et vient se replier vers l'arrière autour des quatre bords libres du plateau arrière 30. Ces replis sont pris en sandwich entre les bords libres du plateau 30 et les faces internes des bords relevés du couvercle avant 20.

On notera également que les bordures relevés du couvercle 20 forment un renflement 22 sur leur face interne dont l'extrémité arrière s'arrête avant l'extrémité de la bordure relevée, sous forme d'un épaulement plat faisant face au plateau 30 et destiné à venir se plaquer contre une portion périphérique de la face avant du plateau 30.

Ce renflement 22 présente, de manière répartie autour du couvercle 20, des logements filetés adaptés pour recevoir des vis de fixation traversant le plateau arrière 30. Ce vissage assure une pression particulièrement forte du renflement 22 sur le plateau 30 avec prise en sandwich de la feuille de Téflon.

La feuille 70 est donc prise en sandwich non seulement entre les extrémités latérales du plateau 30 et la face interne de la bordure relevée du couvercle 20, mais également entre le renflement 22 et la face avant du plateau 30 de sorte que la feuille 70 est soumise, quel que soit l'endroit de la périphérie du plateau 30, à deux forces de pincement perpendiculaires l'une à l'autre.

La feuille 70 recouvre ici l'ensemble du plateau 30 mais elle peut être remplacée par un élément annulaire, par exemple de forme plane et parallèle au plateau 30, ou de forme cylindrique recouvrant seulement la bordure latérale du plateau 30, ou encore de section transversale en équerre pour recouvrir à la fois l'extrémité latérale et la bordure périphérique en face avant du plateau 30.

On note également que le renflement 22 forme avantageusement une gorge, ouverte vers l'arrière et l'intérieur du couvercle 20, recevant de manière complémentaire une nervure périphérique de l'écran 15 pour maintien de celui-ci par pinçage entre le renflement 22 et le plateau 30.

Les présentes pièces 20 et 30 sont réalisées en verre, ce qui leur donne une grande rigidité ainsi qu'une transparence parfaite. Ce verre inclut avantageusement des particules noyées aptes à former une barrière vis à vis d'interférences qui pourraient brouiller les images affichées ou divers signaux de commande décrits ci-après. Il est possible de réaliser l'enceinte en plusieurs pièces dont la seule partie couvrant la face avant de l'écran 15 est réalisée transparente.

L'enceinte peut également être réalisée en plastique, par exemple en Makrolon DP1-1262 commercialisé par BAYER, ou en NORYL.

Le module électronique 40 est relié à l'écran 15 par une liaison 80 simplement disposée dans la cavité du bossage 32.

Le module 40 est également prolongé par une deuxième liaison, double, parcourant la face avant du plateau 30 et la bordure relevée du couvercle 20, jusqu'à déboucher dans l'espace interne de l'enceinte en portant à cette extrémité un capteur de pression 160. Cette double liaison forme une deuxième ligne de transmission qui débouche sur la face avant du couvercle 20 et porte un capteur vocal 150.

Le capteur de pression 160 envoie un signal de mesure au module électronique 40 qui compare alors la pression mesurée à une valeur seuil autorisée pour la pression interne. Cette pression interne autorisée est, dans le présent exemple, une pression nettement supérieure à la pression atmosphérique. En effet, l'enceinte étanche est surpressurisée puis fermée hermétiquement dans une étape préliminaire, et le module électronique surveille la valeur de cette surpression au cours du temps.

En cas de fuite de l'enceinte étanche, c'est à dire en cas de perte d'étanchéité entre l'atmosphère ambiante stérile et les parties internes de l'enceinte non stériles, une perte de pression interne, détectée de suite par le module électronique 40, comme un franchissement à la baisse de la valeur seuil, se traduit par l'affichage par le module 40, sur l'écran 15, d'une alerte visuelle indicatrice de cette perte de pression.

Selon une variante de l'invention, l'enceinte peut également être soumise à une sous pression initiale, le module 40 alertant l'utilisateur lorsque cette sous pression s'atténue de façon non souhaitée.

Le présent dispositif est muni d'un canal de communication fluidique 170 reliant la cavité interne du bossage 32 à l'extrémité du manche de maintien 35. Ce canal 170 est bouché à son extrémité par une valve anti-retour.

Après assemblage de l'enceinte, le manche 35 est engagé dans un dispositif apte à injecter dans le canal 170 un gaz jusqu'à obtenir une pressurisation suffisante de l'enceinte. Une fois l'enceinte séparée de ce dispositif de pressurisation, celle-ci maintient sa pression interne grâce aux dispositions qui viennent d'être décrites avec une fiabilité particulièrement élevée.

Le présent ensemble d'affichage peut être stérilisé plusieurs fois, sans perte de la surpression, aussi bien par immersion que par un procédé à base d'irradiation tel que le procédé connu « STERRAD ».

Un matériel de type « STERRAD » permet une stérilisation à basse température (environ 45°) pour un gaz plasma de type peroxyde d'hydrogène et application d'une radio-fréquence ou de rayons gamma. La stérilisation peut également être réalisée par exemple avec un produit désinfectant standard de type « SIDEC ».

L'ensemble ainsi fermé et étanche peut donc être utilisé plusieurs fois en salle d'opération avec une stérilisation entre chaque utilisation. A chaque utilisation, le personnel d'intervention peut vérifier sur l'écran que l'alerte de perte de pression n'est pas présente et obtenir ainsi une garantie de la parfaite étanchéité de l'enceinte, et donc de sa stérilité.

L'écran est placé à quelques centimètres au-dessus du corps du patient, par exemple au-dessus des mains du chirurgien, de sorte que le praticien ne doit accommoder son oeil que sur une distance réduite, ce qui limite sa fatigue visuelle.

Le présent module 40 électronique analyse en outre les signaux de sortie du capteur 170 pour commander selon ces signaux différentes fonctions du présent écran 15, et également transmettre des commandes à d'autres dispositifs tels qu'un outil endoscopique non représenté.

Pour transmettre ou recevoir les images à afficher, le module 40 présente des liaisons filaires traversant la partie arrière 32 de l'enceinte et débouchant sur la surface externe arrière de cette partie. Ces liaisons filaires portent à leur extrémité des contacts électriques 50 qui sont hermétiquement liés avec le matériau constitutif de la partie 32, pour assurer une forte étanchéité à leur niveau. Cette connexion étanche à contacts est ici une connexion étanche de type billes de contact.

Plus précisément, les liaisons électriques parcourent longitudinalement le manche 35 et les contacts métalliques 50 sont répartis le long du manche 35.

Le support 100 est un support articulé, à bras et rotules, dont la portion d'extrémité forme une cavité 102 complémentaire du bras 35, pour recevoir le bras 35 et maintenir ainsi l'ensemble d'affichage dans la position souhaitée. Les rotules du support 100 sont préférentiellement prévues étanches pour une stérilisation aisée, notamment par immersion, de ce support.

Cette cavité 102 du support est également pourvue sur sa face interne de contacts métalliques venant se placer contre les contacts du bras 50. Ces contacts sont prolongés par des liaisons électriques parcourant l'ensemble des bras et rotules du support 100 et adaptés pour permettre les mouvements des rotules.

Ainsi, l'enfichage du bras 35 dans la cavité correspondante 102 du support produit à la fois un maintien mécanique de l'ensemble d'affichage 10 et une liaison d'échange de signaux entre le module électronique 40 et des aménagements électroniques externes, tels que les outils et caméras endoscopiques non représentés.

On prévoit ici une goupille 110 traversant une paroi latérale de la cavité 102 et venant se loger dans un alésage correspondant du bras 35. Cette goupille 110 maintient donc le bras 35 contre une extraction accidentelle de la cavité 102 et contre une rotation du bras 35 dans la cavité 102.

Les contacts et liaisons du support avec l'ensemble d'affichage véhiculent également dans le présent exemple une énergie électrique d'alimentation du module 40 et de l'écran 15.

Selon une variante, le module 40 peut également inclure une batterie chargée avant assemblage et pressurisation de l'ensemble d'affichage 10.

Selon une autre variante, le module 40 se prolonge par au moins une liaison électrique dont une extrémité porte une antenne pour émission, réception, ou les deux, d'ondes électromagnétiques. Les liaisons peuvent également porter un émetteur lumineux, un capteur lumineux ou les deux. Les capteurs et émetteurs de l'ensemble d'affichage sont par exemple des capteurs et/ou émetteurs infrarouges. Ces éléments d'émission ou de réception électromagnétique ou lumineux sont préférentiellement noyés dans le matériau constitutif de la pièce 30, en un emplacement proche de la surface externe de la pièce 30. Ainsi, on place préférentiellement des moyens de réception électromagnétique comprenant une antenne de réception en un emplacement où l'enceinte présente entre cette antenne et l'extérieur une épaisseur plus faible que l'épaisseur moyenne de l'enceinte.

L'énergie d'alimentation de l'écran 15 et du module 40 est alors préférentiellement transmise sous la forme de telles ondes électromagnétiques ou lumineuses.

Dans ces variantes, les parois internes de la cavité 102 présentent avantageusement des récepteurs/émetteurs électromagnétiques ou lumineux correspondant chacun aux émetteurs/récepteurs de l'ensemble d'affichage.

De tels éléments de connexion sans contact peuvent également être des éléments de type à induction, pour la transmission de signaux ou pour la transmission de l'énergie d'alimentation.

A la lumière des éléments ci-dessus décrits, l'enceinte et les éléments qu'elle entoure forment un ensemble qui est stérilisable à l'infini, c'est à dire sans considération d'un nombre limite de stérilisations, fixé à l'avance. En effet, l'éventuel arrêt des stérilisations est signalé par l'alerte donnée grâce au capteur de pression. Le ou les capteurs de pression interne garantissent une stérilisation efficace et répétitive et garantissent, en l'absence d'alerte, le parfait état de fonctionnement et d'étanchéité du dispositif, notamment avant et après stérilisation. L'ensemble décrit est utilisable dans des salles stériles de toute nature, médicales ou non, telles que bloc opératoire, laboratoire, ou encore salles où l'on travaille sur des bactéries dont le développement peut être modifié par la présence d'objets non stériles.

## Revendications

1. Dispositif pour observer un champ chirurgical, comprenant un moniteur vidéo plat (15, 40) et une enceinte (20, 30) qui est stérilisable et qui forme barrière de stérilité fermée de manière étanche autour du moniteur (15, 40), **caractérisé en ce que** l'enceinte étanche (20, 30) est rigide, qu'elle comporte une sous-pression ou une surpression interne maintenue par son étanchéité et qu'elle incorpore un capteur de pression interne (160) coopérant avec des moyens d'alerte en cas de disparition de l'étanchéité, le dispositif assemblé étant ainsi utilisable avec sécurité dans des salles stériles de toute nature.

2. Dispositif selon la revendication 1, **caractérisé en ce que** l'enceinte (20, 30) est munie d'une liaison électrique la traversant de l'intérieur vers l'extérieur et formant à son extrémité extérieure un contact électrique (50) lié à l'enceinte (20, 30) de manière étanche.

3. Dispositif selon l'une des revendications précédentes, **caractérisé en ce qu'**il comprend des moyens de réception électromagnétique liés électriquement au moniteur (15, 40), prévus pour recevoir une énergie électromagnétique d'alimentation du moniteur (15, 40).

4. Dispositif selon l'une quelconque des revendications précédentes, **caractérisé en ce qu'**il comprend des moyens de réception électromagnétique comprenant une antenne de réception disposée en un emplacement où l'enceinte (20, 30) présente entre cette antenne et l'extérieur une épaisseur plus faible que l'épaisseur moyenne de l'enceinte (20, 30).

5. Dispositif selon l'une quelconque des revendications 3 ou 4, **caractérisé en ce que** les moyens de réception électromagnétique sont également prévus pour émettre des signaux électromagnétiques vers un dispositif de réception extérieure à l'enceinte.

6. Dispositif selon l'une quelconque des revendications précédentes, **caractérisé en ce qu'**il comprend des moyens d'émission ou de réception d'ondes infrarouges.

7. Dispositif selon la revendication 6, **caractérisé en ce que** les moyens d'émission ou de réception d'ondes infrarouges sont prévus pour collecter une énergie lumineuse d'alimentation du moniteur (15, 40).

8. Dispositif selon l'une des revendications précédentes, **caractérisé en ce qu'**il comporte un support (100) d'enceinte, l'enceinte (20, 30) et le support (100) formant des moyens d'enfichage (35, 102) complémentaires pour maintenir l'enceinte (20, 30) sur le support (100), ces moyens d'enfichage (35, 102) portant des moyens de connexion complémentaires de sorte que l'enfichage provoque également une liaison d'échange entre l'enceinte (20, 30) et le support (100).

9. Dispositif selon la revendication 8, **caractérisé en ce que** l'enceinte (20, 30) forme un bras (35) destiné à être inséré dans une cavité complémentaire (102) du support (100), et **en ce que** le bras (35) et la cavité (102) comportent des aménagements de verrouillage (100) du bras (35) dans la cavité (102) s'opposant à une rotation du bras (35) dans la cavité (102).

10. Dispositif selon l'une des revendications 1 à 9, **caractérisé en ce que** l'enceinte (20, 30) est réalisée en verre.

11. Dispositif selon l'une quelconque des revendications précédentes, **caractérisé en ce que** l'enceinte comporte au moins deux éléments (20, 30) et un joint en Téflon (70) disposé entre au moins deux parties respectives de ces deux éléments (20, 30).

## Patentansprüche

1. Vorrichtung zum Beobachten eines Operationsfelds, das einen flachen Videomonitor (15, 40) und eine Hülle (20, 30), die sterilisierbar ist und die eine dicht abgeschlossene Sterilitätsschranke um den Monitor (15, 40) bildet, umfasst, wobei die dichte Hülle (20, 30) steif ist, **dadurch gekennzeichnet, dass** sie einen inneren Unterdruck oder Überdruck aufweist, der durch ihre Dichtigkeit aufrechterhalten wird, und dass sie einen innenliegenden Drucksensor (160) einschließt, der mit Mitteln zum Geben eines Alarms im Falle eines Verlusts der Dichtigkeit zusammenarbeitet, wodurch die zusammengesetzte Vorrichtung in sterilen Sälen aller Art mit Sicherheit einsetzbar ist.

2. Vorrichtung nach Anspruch 1, **dadurch gekennzeichnet, dass** die Hülle (20, 30) mit einem elektrischen Anschluss ausgestattet ist, der sie von innen nach außen durchstößt und der an seinem äußeren Ende einen elektrischen Kontakt (50) bildet, der abdichtend mit der Hülle (20, 30) verbunden ist.

3. Vorrichtung nach einem der vorhergehenden Ansprüche, **dadurch gekennzeichnet, dass** sie Mittel zum elektromagnetischen Empfang umfasst, die elektrisch mit dem Monitor (15, 40) verbunden und dafür vorgesehen sind, eine elektromagnetische Versorgungsenergie des Monitors (15, 40) zu empfangen.

4. Vorrichtung nach einem beliebigen der vorhergehenden Ansprüche, **dadurch gekennzeichnet, dass** sie Mittel zum elektromagnetischen Empfang umfasst, die eine Empfangsantenne umfassen, die an einer Stelle angeordnet ist, an der die Hülle (20, 30) zwischen dieser Antenne und dem Äußeren eine geringere Dicke als die mittlere Dicke der Hülle (20, 30) aufweist.

5. Vorrichtung nach einem beliebigen der Ansprüche 3 oder 4, **dadurch gekennzeichnet, dass** die Mittel zum elektromagnetischen Empfang auch zum Senden von elektromagnetischen Signalen zu einer außerhalb der Hülle liegenden Empfangsvorrichtung vorgesehen sind.

6. Vorrichtung nach einem beliebigen der vorhergehenden Ansprüche, **dadurch gekennzeichnet, dass** sie Mittel zum Empfang oder zum Senden von Infrarotwellen umfasst.

7. Vorrichtung nach Anspruch 6, **dadurch gekennzeichnet, dass** die Mittel zum Empfang oder zum Senden von Infrarotwellen dafür vorgesehen sind, Lichtenergie zur Versorgung des Monitors (15, 40) zu sammeln.

8. Vorrichtung nach einem der vorhergehenden Ansprüche, **dadurch gekennzeichnet, dass** sie einen Halter (100) der Hülle umfasst, wobei die Hülle (20, 30) und der Halter (100) komplementäre Steckverbindungsmittel (35, 102) ausbilden, um die Hülle (20, 30) auf dem Halter (100) zu halten, wobei diese Steckverbindungsmittel (35, 102) komplementäre Anschlussmittel tragen, derart, dass das Zusammenstecken auch eine Austauschverbindung zwischen der Hülle (20, 30) und dem Träger (100) bewirkt.

9. Vorrichtung nach Anspruch 8, **dadurch gekennzeichnet, dass** die Hülle (20, 30) einen Arm (35) ausbildet, der dafür vorgesehen ist in eine komplementäre Aussparung (102) des Halters (100) eingeführt zu werden und dadurch, dass der Arm (35) und die Aussparung (102) Einrichtungen zum Verriegeln (100) des Arms (35) in der Aussparung (102) umfassen, die sich einer Drehung des Arms (35) in der Aussparung (102) wiedersetzen.

10. Vorrichtung nach einem der Ansprüche 1 bis 9, **dadurch gekennzeichnet, dass** die Hülle (20, 30) aus Glas gefertigt ist.

11. Vorrichtung nach einem beliebigen der vorhergehenden Ansprüche, **dadurch gekennzeichnet, dass** die Hülle wenigstens zwei Elemente (20, 30) und eine Teflondichtung (70), die zwischen wenigstens zwei jeweiligen Teilen dieser zwei Elemente (20, 30) angeordnet ist, umfasst.

## Claims

1. Device for observing a surgical field, comprising a flat video monitor (15, 40) and a chamber (20, 30) which is sterilizable and which forms a sterility barrier closed in a sealed manner around the monitor (15, 40), the sealed chamber (20, 30) being rigid, **characterized in that** the sealed chamber has an internal underpressure or overpressure maintained by its sealing, and **in that** it incorporates an internal pressure sensor (160) cooperating with alarm means in the event of the sealing disappearing, the assembled device thus being able to be used with safety in sterile areas of any type.

2. Device according to Claim 1, **characterized in that** the chamber (20, 30) is provided with an electrical connection passing through it from the inside to the outside and forming at its outer end an electrical contact (50) connected to the chamber (20, 30) in a sealed manner.

3. Device according to one of the preceding claims, **characterized in that** it comprises means for electromagnetic reception which are connected electrically to the monitor (15, 40) and are intended to receive electromagnetic energy for powering the monitor (15, 40).

4. Device according to any one of the preceding claims, **characterized in that** it comprises means for electromagnetic reception comprising a reception antenna arranged at a location where the chamber (20, 30) has, between this antenna and the outside, a thickness which is less than the average thickness of the chamber (20, 30).

5. Device according to either one of Claims 3 and 4, **characterized in that** the means for electromagnetic reception are also intended to send electromagnetic signals to a reception device outside the chamber.

6. Device according to any one of the preceding claims, **characterized in that** it comprises means for emitting or receiving infrared waves.

7. Device according to Claim 6, **characterized in that** the means for emitting or receiving infrared waves are intended to collect light energy for powering the monitor (15, 40).

8. Device according to one of the preceding claims, **characterized in that** it comprises a support (100) for the chamber, said chamber (20, 30) and said support (100) forming complementary engagement means (35, 102) for maintaining the chamber (20, 30) on the support (100), these engagement means (35, 102) having complementary connection means so that the engagement also effects an exchange connection between the chamber (20, 30) and the support (100).

9. Device according to Claim 8, **characterized in that** the chamber (20, 30) forms an arm (35) intended to be inserted in a complementary cavity (102) of the support (100), and **in that** the arm (35) and the cavity (102) comprise arrangements for locking (105) the arm (35) in the cavity (102) and opposing a rotation of the arm (35) in the cavity (102).

10. Device according to one of Claims 1 to 9, **characterized in that** the chamber (20, 30) is made of glass.

11. Device according to any one of the preceding claims, **characterized in that** the chamber comprises at least two elements (20, 30) and a Teflon seal (70) arranged between at least two respective parts of these two elements (20, 30).
